# EUROPEAN PATENT APPLICATION

(11) **EP 3 505 319 A2**
(43) Date of publication of application: **03.07.2019**
(21) Application number: 18213184.7
(22) Date of filing: 17.12.2018
(51) Int. Cl.: B29B 7/84, B29B 7/18, B29B 7/24, B29B 7/26, B29B 7/42, B29B 7/72, B29B 7/74, B29B 7/82, B29C 48/395, B29C 48/285, B29C 48/76, B29C 48/92, G01N 33/44

(54) **RUBBER EXTRUDING DEVICE AND RUBBER EXTRUDING METHOD**

(30) Priority: 27.12.2017 JP 2017251290
(71) Applicant: Sumitomo Rubber Industries, Ltd., Kobe-shi, Hyogo-ken 651-0072 (JP)
(72) Inventor: SAKAMOTO, Masayuki, Kobe-shi, Hyogo-ken, 651-0072 (JP)
(74) Representative: Manitz Finsterwald Patent- und Rechtsanwaltspartnerschaft mbB

(57) **Abstract**

Rubber extruding device 1 including a closed type mixer 2 for kneading unvulcanized rubber material G, an extruder 3 which receives kneaded rubber Ga from the mixer 2 and extrudes, while further kneading, as an extruded rubber strip Gb, and a conveyor 4 for carrying the unvulcanized rubber material G to the mixer 2, whereas the extruder 3 further includes at least one deaerator 14 for sucking up a gas in the extruder 3 and may optionally further include an ethanol sensor 15 for detecting ethanol, e.g., ethanol concentration, with the deaerator 14 and the ethanol sensor 15 being provided on the casing main body 12A of the extruder 3.

## Description

### BACKGROUND ART

### Field of the Invention

The present invention relates to rubber extruding devices, and more particularly to a tuber extruding device for extruding unvulcanized rubber material, using a mixer and an extruder.

### Description of the Related Art

As rubber extruding devices for kneading unvulcanized rubber material, mixers kneading unvulcanized rubber material in substantially closed chambers as well as extruders having screw shafts for kneading and extruding unvulcanized rubber material have been known. As mixers, Banbury (registered trademark) mixers, for example, have been well known, and which are suitable to plasticize unvulcanized rubber material and to disperse addition agents into the material uniformly while suppressing scattering of the addition agents to outside. The extruders, for example, are suitably used for extruding rubber material continuously as a strip having a predetermined cross-section.

For example, the following Patent document 1 discloses a rubber extruding device which includes a mixer and an extruder that receives rubber material kneaded by the mixer previously and that further kneads and extrudes the same. The rubber extruding device can knead unvulcanized rubber material uniformly to promote chemical reaction in the material, resulting in improving the quality of kneaded rubber.

[Patent document 1] Japanese Unexamined Patent Application Publication 2016-141056

### SUMMARY OF THE DISCLOSURE

When unvulcanized rubber material is kneaded, gases, e.g., ethanol can be generated. Through various experiments, it has been found removing ethanol gas generated during the kneading process of unvulcanized rubber material promotes chemical reaction for the rubber material.

Unfortunately, since the rubber extruding device as disclosed in Patent document 1 has no means for removing gases such as ethanol generated during the kneading process, there has been room for improvement promoting chemical reaction in unvulcanized rubber material.

In view of the above problems in the conventional art, the present disclosure has an object to provide a rubber extruding device and a rubber extruding method which may improve quality of unvulcanized rubber kneaded and extruded.

According one aspect of the present disclosure, a rubber extruding device for kneading and extruding unvulcanized rubber material, the rubber extruding device includes a mixer for kneading unvulcanized rubber material in a substantially closed mixing chamber, and an extruder including a rotatable screw shaft for kneading further the unvulcanized rubber material received from the mixer and for extruding the same, and at least one deaerator for sucking up a gas in the extruder.

In another aspect of the disclosure, the extruder further may include a sensor for detecting ethanol in the extruder.

In another aspect of the disclosure, the sensor may be arranged at a downstream side of the at least one deaerator in a rubber extruding direction.

In another aspect of the disclosure, the at least one deaerator may include a plurality of deaerators.

In another aspect of the disclosure, the extruder may further include a casing housing the screw shaft, and a temperature controller for adjusting a temperature of the casing and/or the screw shaft at a predetermined temperature range.

In another aspect of the disclosure, the predetermined temperature range may be in a range of from 120 to 165 degrees C.

In another aspect of the disclosure, a method for kneading and extruding unvulcanized rubber material, the method includes a first kneading step of kneading unvulcanized rubber material in a substantially closed mixing chamber of a mixer, and a second kneading step of kneading further the unvulcanized rubber material received from the mixer using an extruder having a rotatable screw shaft while sucking up a gas in the extruder.

In another aspect of the disclosure, the second kneading step may further include a step of detecting a concentration of ethanol in the extruder; and a step of changing an operation speed of the extruder based on the concentration of ethanol.

In another aspect of the disclosure, the second kneading step may further include a step of detecting a concentration of ethanol in the extruder; and a step of changing a temperature of the extruder based on the concentration of ethanol.

### BRIEF DESCRIPTION OF THE DRAWINGS

FIG. 1 is a conceptual cross-sectional view of a rubber extruding device in accordance with an embodiment of the present disclosure;
FIG. 2 is a cross-sectional view of a mixer;
FIG. 3 is a cross-sectional view of an extruder;
FIG. 4 is a flowchart for explaining a rubber extruding method in accordance with an embodiment of the disclosure; and
FIG. 5 is a flowchart for explaining a second kneading step.

### DESCRIPTION OF THE PREFERRED EMBODIMENTS

An embodiment of the present invention will be explained below with reference to the accompanying drawings.

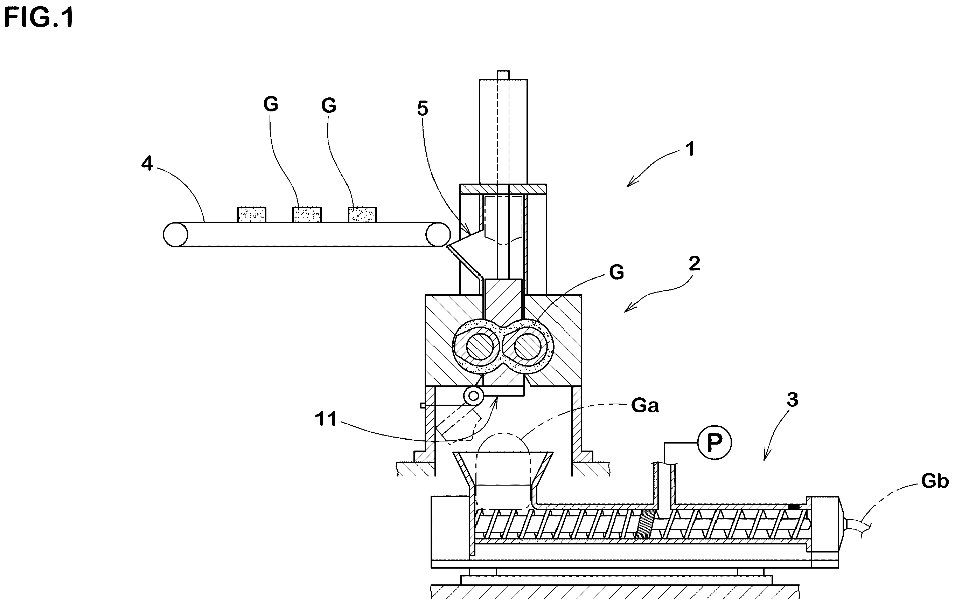

FIG. 1 is a conceptual cross-sectional view of a rubber extruding device 1 in accordance with an embodiment of the present disclosure. As illustrated in FIG. 1, the rubber extruding device 1 is configured to knead and extrude unvulcanized rubber material.

In this embodiment, the rubber extruding device 1 includes a closed type mixer 2 for kneading unvulcanized rubber material G, an extruder 3 which receives kneaded rubber Ga from the mixer 2 and extrudes, while further kneading, as an extruded rubber strip Gb, and a conveyor 4 for carrying the unvulcanized rubber material G to the mixer 2.

As the mixer 2, a Banbury mixer and an intermeshing mixer can suitably be employed. These mixers can knead unvulcanized rubber material G in a substantially closed mixing chamber.

The mixer 2 is configured to plasticize unvulcanized rubber material G and to disperse addition agents into the material G uniformly while suppressing scattering of the addition agents outside, resulting in improving the quality of kneaded rubber Ga.

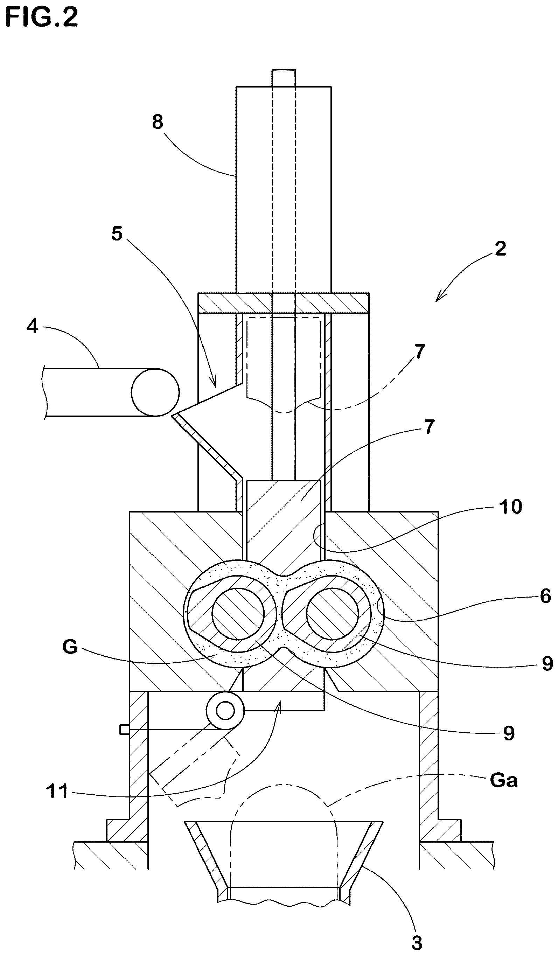

FIG. 2 illustrates a cross-sectional view of the mixer 2.

As illustrated in FIG. 2, in this embodiment, the mixer 2 includes a feeding port 5 for unvulcanized rubber material G, a mixing chamber 6 for receiving unvulcanized rubber material G put through the feeding port 5, a ram weight 7 for pressuring unvulcanized rubber material G in the mixing chamber 6, and an elevating device 8 for moving the ram weigh 7 up and down.

In the mixing chamber 6, a pair of parallelly arranged mixing rotors 9 are arranged. By being rotated the mixing rotors 9, the unvulcanized rubber material G and addition agents are kneaded together in the mixing chamber 6, resulting in plasticizing the unvulcanized rubber material G as well as dispersing the addition agent in the material G. Note that the addition agents may be fed through the feeding port 5 with the unvulcanized rubber material G, or through another feeding port (not illustrated).

Each of the mixing rotors 9, for example, is configured to have a base shaft and a spirally extending agitating blade provided on the base shaft. Preferably, the mixing rotors 9 are rotated by an electric motor (not illustrated) in the same and/or reverse directions as each other.

The mixing chamber 6, for example, has a gourd-shaped cross-section which includes two combined circular surfaces each having the same centers as the respective one of the mixing rotors 9. The gourd-shaped cross-section has a constriction region to which a longitudinal passage 10 extending from the feeding port 5 is connected, for example.

In this embodiment, the ram weight 7 is movably arranged in the longitudinal passage 10. At the lowermost location of the ram weight 7, the ram weight 7 can close the mixing chamber 6 such that the mixing chamber 6 is to be a substantially closed space, thus, pressuring unvulcanized rubber material G fed in the mixing chamber 6. As a result, the ram weight 7 may improve mixing efficiency and kneading efficiency of the mixing rotors 9. Note that as the elevating device 8, for example, a conventional fluid pressure cylinder may be employed.

Preferably, the rubber G kneaded by the mixer 2 is discharged, through an openable outlet port 11 provided below the mixing chamber 6, as a bulk shaped rubber Ga toward the extruder 3 directly. In this embodiment, since conveying time for the rubber Ga from the mixer 2 to the extruder 3 is unnecessary, the rubber Ga, in succession, can be kneaded by the extruder 3 without suspending the chemical reaction in the rubber Ga.

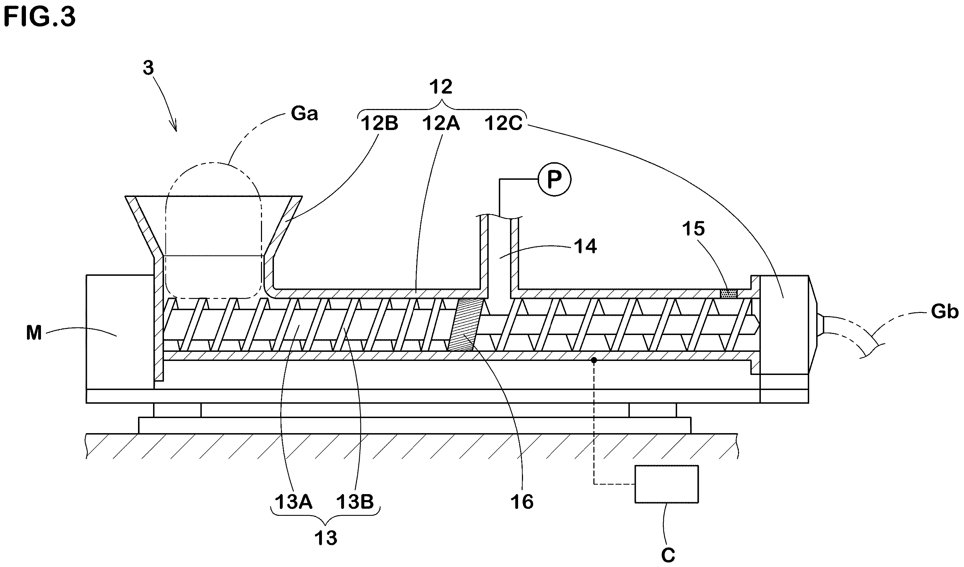

FIG. 3 illustrates a cross-sectional view of the extruder 3. As illustrated in FIG. 3, in this embodiment, the extruder 3 includes a casing 12, and a screw shaft 13 disposed in the casing 12. The extruder 3 can knead the rubber Ga and extrude it continuously as an extruded rubber body Gb having a predetermined cross-section.

In this embodiment, the casing 12 includes a casing main body 12A housing the screw shaft 13, a hopper 12B for receiving the kneaded rubber Gs from the mixer 2, and a head 12C for extruding the extruded rubber body Gb.

The screw shaft 13 can knead the rubber Ga in the casing 12 further, and then extrudes it. In this embodiment, the screw shafts 13 includes a base shaft 13A and a spiral blade 13B protruding radially outwardly of the base shaft 13A.

The base shaft 13A extends in a straight shape along the longitudinal direction of the casing 12. The spiral blade 13B, for example, extends over the entire length of the base shaft 13A in the axial direction. The spiral blade 13B can extrude the rubber Ga in the casing 12 while kneading. Preferably, the screw shaft 13 is driven by a drive controller M arranged outside the casing 12.

In this embodiment, a temperature controller C is provided for adjusting the temperature of the casing 12 and/or the screw shaft 13 at a predetermined temperature range. In some preferred embodiments, the temperature controller C controls the casing 12 and/or the screw shaft 13 at a temperature ranging from 120 to 165 degrees C. The temperature controller C can accelerate chemical reaction for the rubber Ga, improving the quality of rubber Ga.

In this embodiment, the extruder 3 further includes at least one deaerator 14 for sucking up a gas in the extruder 3. Optionally, the extruder 3 may further include an ethanol sensor 15 for detecting ethanol, e.g., ethanol concentration. In this embodiment, the deaerator 14 and the ethanol sensor 15 are provided on the casing main body 12A.

Preferably, the deaerator 14 is in communication with a vacuum device P to generate a negative pressure in the interior of the extruder 3. Thus, the deaerator 14 can remove gasses, e.g., ethanol gas and the like, generated from the rubber Ga during the kneading in the extruder 3, accelerating chemical reaction in the rubber Ga, improving the quality thereof.

In this embodiment, the screw shaft 13 includes a dam section 16 at an upstream location of the deaerator 14 in the rubber extruding direction. The dam section 16 restricts the amount of rubber G passing therethrough to low. In the extruder 3, the deaerator 14 can suck up gases effectively because the amount of the rubber Ga passing the deaerator is limited by the dam section 16.

In this embodiment, the extruder 3 is provided with a single deaerator 14. In another aspect, the extruder 3 may include a plurality of deaerators 14. Such an extruder 3 having a plurality of deaerators 14 may generate a large negative pressure in the interior of the extruder 3, thus sucking up gases including ethanol generated from the rubber Ga effectively to remove.

Preferably, the ethanol sensor 15 is arranged at a downstream side of the deaerator 14 in the rubber extruding direction. Thus, the ethanol sensor 15 can detect concentration of ethanol remaining in the rubber Ga after which gasses are sucked up by the deaerator 14. Based on the detected concentration of ethanol, the degree of chemical reaction in the rubber, e.g., kneaded condition of the rubber Ga, can be obtained accuracy.

Preferably, the ethanol sensor 15 is located proximate to the head 12C. In some preferred embodiments, the ethanol sensor 15 is provided on the head 12C. In this aspect, the ethanol sensor 15 can detect the ethanol generated from the rubber Ga just before being extruded, thereby capable of observing a condition of extruded rubber Gb accuracy as well as kneaded condition of the rubber Ga.

As illustrated in FIG. 1. in this embodiment, the conveyor 4 extends from a storage space (not illustrated) for unvulcanized rubber material G to the feeding port 5 of the mixer 2 to carry the unvulcanized rubber material G smoothly. As the conveyor 4, for example, various kinds of belt conveyor can be employed.

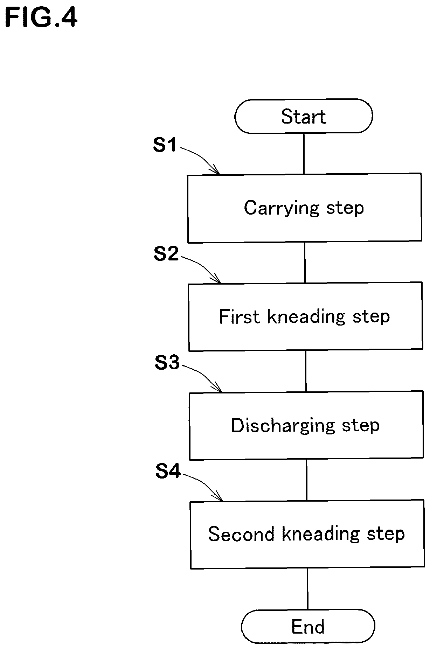

Next, a rubber extruding method in accordance with an embodiment of the disclosure will be explained based on FIGS. 1 to 4. FIG. 4 illustrates a flowchart of the rubber extruding method according to the embodiment. As illustrated in FIG. 4, the method is directed to a method for extruding unvulcanized rubber material G while kneading the same.

In this embodiment, the method includes a carrying step S1 of carrying unvulcanized rubber material G from a storage space to the mixer 2. The carrying step S1, for example, carries a bulk shaped unvulcanized rubber material G by the conveyor 4. Preferably, the carrying step S1 may intermittently be conducted in association with a first kneading step S2 which will be described later.

In this embodiment, next, the method includes the first kneading step S2 of kneading the unvulcanized rubber material G in the mixer 2. In the first kneading step S2, the ram weight 7 is moved upwardly to open the mixing chamber 6, and then unvulcanized rubber material G and one or more addition agents are put into the mixing chamber 6 through the feeding port 5. Next, the ram weight 7 is moved downwardly so as to close the mixing chamber 6 in a sealed condition, thus, pressuring unvulcanized rubber material G. Next, the mixing rotors 9 are rotated to knead the unvulcanized rubber material G and the addition agents in the mixing chamber 6, and thus the unvulcanized rubber material G is plasticized while dispersing the addition agents.

In the first kneading step S2, the addition agents are dispersed in the unvulcanized rubber material G uniformly while suppressing scattering of the addition agents to outside, thus accelerating chemical reaction in the unvulcanized rubber material G.

In this embodiment, next, a discharging process S3 of discharging the rubber G kneaded by the mixer 2 from the mixer 2 is conducted. In the discharging step S3, for example, the mixer 2 discharges a bulk-shaped rubber Ga to the extruder 3 by opening the outlet port 11, e.g., making it a fall.

In the discharging step S3, since conveying time for the rubber Ga from the mixer 2 to the extruder 3 is unnecessary, the rubber Ga, in succession, can be kneaded by the extruder 3 without suspending the chemical reaction in the rubber Ga.

In this embodiment, a second kneading step S4 is conducted in such a manner that the rubber Ga kneaded in the first kneading step S2 is further kneaded and extruded by the extruder 3 having the screw shaft. In the second kneading step S4, gasses generated from the rubber Ga in the extruder 3 is sucked up while kneading. In the second kneading step S4, the rubber Ga is preferably extruded as an extruded rubber body Gb having a predetermined cross-section continuously.

By the second kneading step S4, gasses including ethanol generated from the rubber Ga during kneading can be removed, the chemical reaction in the rubber Ga is accelerated further, improving the rubber Ga in quality.

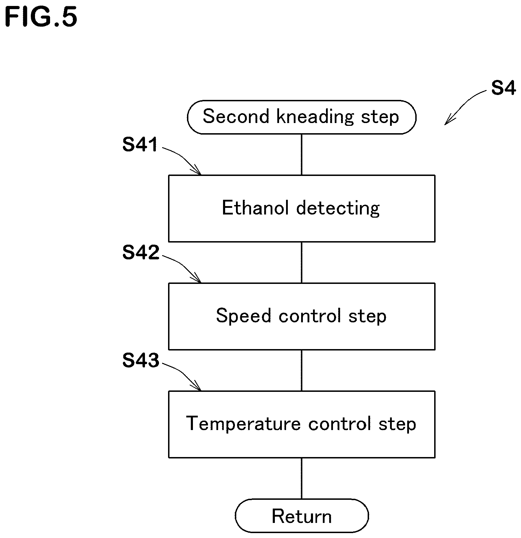

FIG. 5 illustrates a flowchart for explaining the details of the second kneading step S4. As illustrated in FIG. 5, in this embodiment, the second kneading step S4, at first, an ethanol detecting step S41 of detecting ethanol concentration in the extruder 3 by the ethanol sensor 15 is conducted. By the ethanol detecting step S41, the concentration of ethanol generated from the rubber Ga can be detected, and thus the degree of chemical reaction in the rubber, e.g., the kneaded condition of the rubber Ga, can be obtained accuracy.

In the second kneading step S4, next, a speed control step S42 of changing an operation speed of the extruder 3 based on the concentration of ethanol detected. In the speed control step, S42, for example, when the concentration of ethanol detected is high compared with a predetermined threshold, it is determined that the chemical reaction in the rubber Ga is surely achieved, and thus increasing the operation speed of the extruder 3 to improve productivity.

In the second kneading step S4, next, a temperature control step S43 of changing a temperature of the extruder 3 based on the concentration of ethanol detected. In the temperature control step S43, for example, when the concentration of ethanol detected is low compared with a predetermined threshold, it is determined that the chemical reaction in the rubber Ga is not enough, and thus increasing the temperature of the extruder 3 to accelerate the chemical reaction in the rubber Ga further.

Note that in the second kneading step S4, it is not necessary to conduct both the speed control steps S42 and the temperature control step S43, but only either one of the steps can be conducted.

While the particularly preferable embodiments in accordance with the present disclosure have been described in detail, the present disclosure is not limited to the illustrated embodiments, but can be modified and carried out in various aspects.

## Claims

1. A rubber extruding device for kneading and extruding unvulcanized rubber material, the rubber extruding device comprising:
a mixer for kneading unvulcanized rubber material in a substantially closed mixing chamber; and
an extruder comprising a rotatable screw shaft for kneading further the unvulcanized rubber material received from the mixer and for extruding the same, and at least one deaerator for sucking up a gas in the extruder.

2. The rubber extruding device according to claim 1, the extruder further comprising a sensor for detecting ethanol in the extruder.

3. The rubber extruding device according to claim 2, wherein
the sensor is arranged at a downstream side of the at least one deaerator in a rubber extruding direction.

4. The rubber extruding device according to any one of claims 1 to 3, wherein
the at least one deaerator comprises a plurality of deaerators.

5. The rubber extruding device according to any one of claims 1 to 4,
the extruder further comprising a casing housing the screw shaft, and
a temperature controller for adjusting a temperature of the casing and/or the screw shaft at a predetermined temperature range.

6. The rubber extruding device according to claim 5, wherein
the predetermined temperature range is in a range of from 120 to 165 degrees C.

7. A method for kneading and extruding unvulcanized rubber material, the method comprising:
a first kneading step of kneading unvulcanized rubber material in a substantially closed mixing chamber of a mixer; and
a second kneading step of kneading further the unvulcanized rubber material received from the mixer using an extruder having a rotatable screw shaft while sucking up a gas in the extruder.

8. The method for kneading and extruding unvulcanized rubber material according to claim 7, the second kneading step further comprising:
a step of detecting a concentration of ethanol in the extruder; and
a step of changing an operation speed of the extruder based on the concentration of ethanol.

9. The method for kneading and extruding unvulcanized rubber material according to claim 7 or 8, the second kneading step further comprising:
a step of detecting a concentration of ethanol in the extruder; and
a step of changing a temperature of the extruder based on the concentration of ethanol.
